# EUROPEAN PATENT APPLICATION

(11) **EP 4 802 939 A1**
(43) Date of publication of application: **09.09.2026**
(21) Application number: 24885065.3
(22) Date of filing: 16.10.2024
(51) Int. Cl.: A41D 19/01, A41F 1/06, A61M 25/02

(54) **GLOVE FOR PATIENTS**

(30) Priority: 31.10.2023 ES 202331921 U
(71) Applicant: Bermejo Noguero, Juan Jose, 14200 Cordoba (ES)
(72) Inventor: Bermejo Noguero, Juan Jose, 14200 Cordoba (ES)
(74) Representative: González López-Menchero, Álvaro Luis
(86) International application number: PCT/ES2024/070629
(87) International publication number: WO 2025/093788

(57) **Abstract**

The present invention relates to a glove for patients comprising a mitt (1) that completely covers the fingers and hand, extending up to the wrist, where it is attached to a flexible thick strip (2) connected to a sleeve (3), such that there is a flexible narrow strip (4) at the free end of the sleeve (3), said narrow strip being attached to an adjustable fastening strap (5). Said fastening strap has a superimposed securing tape (6) configured to securely fasten and secure the medical line of the patient.

## Description

### OBJECT OF THE INVENTION

The invention, as stated in the title of the present specification, is a glove for patients. It is an innovation that brings previously unknown advantages to current techniques.

### TECHNICAL SECTOR

The present invention falls within the sector of outerwear, protective clothing and accessories, especially gloves.

### STATE OF THE ART

The present invention arises from the fact that hospitalised patients, when disoriented or uncomfortable, tend to try to remove the intravenous lines from their arm, which can cause them serious harm.

In view of this situation, medical staff opt to tie them to the bed as a precautionary measure, creating even more discomfort for the patient and their relatives.

Accordingly, the present invention consists of a glove for patients that safely and comfortably protects the patient's integrity and prevents them from removing the intravenous lines from their arm, providing peace of mind to both medical staff and relatives by avoiding the need to tie the patient to the bed.

At present, there is no known glove for patients with the same or similar structural and constitutive technical features as those described in this specification as claimed.

### DESCRIPTION OF THE INVENTION

The object of the present invention is the creation of a glove for patients, which provides a notable innovation within its field of application in the current state of the art, the characterising details that make it possible being conveniently included in the final claims that accompany the present description.

The present invention is a glove for patients, which comprises a mitt that completely covers the fingers and hand, extending up to the wrist, where it is attached to a thick strip which is in turn connected to a sleeve. There is a narrow strip at the upper end of the sleeve, said narrow strip being attached to an adjustable fastening strap which has a securing tape to securely fasten and secure the medical line of the patient, preventing it from being accidentally moved or displaced.

This invention solves the problem of having to tie the patient to the bed to prevent them from removing the intravenous lines. Alternatively, the intravenous lines are secured and the patient is prevented from using their hand to remove them, while maintaining the rest of their mobility.

This invention is highly beneficial in providing confidence to both medical staff and relatives, as it eliminates the obligation to restrain the patient to the bed in order to prevent them from removing the intravenous lines.

The glove has a lightweight anatomically shaped mitt, which in use has a convex shape on the outside, similar to a boxing glove, and a slightly concave shape to rest the palm of the hand.

Inside said glove, the fingers can have individual spaces for each of them. Furthermore, it is made of soft, hypoallergenic, padded and breathable materials, which provides a pleasant sensation and prevents any kind of skin irritation or allergy.

The mitt is attached to the sleeve by means of the flexible thick strip that covers the contour of the wrist and allows movement. The sleeve extends, covering the forearm until it reaches near the elbow, where it is attached to a thin strip that is also attached with an adjustable fastening strap that adjusts to the forearm and has a press stud for added security.

This adjustable fastening strap is attached to a securing tape which ensures the secure fastening of the medical line of the patient.

To use the glove, the mitt is placed on the patient's hand, making sure, if necessary, to place each finger in its corresponding space.

The sleeve is then slipped over the forearm and the fastening strap is adjusted and secured with the press stud for secure fastening.

Lastly, the securing tape is used to ensure the stability of the medical lines of the patient, preventing any kind of involuntary movement or displacement.

### EXPLANATION OF THE DRAWINGS

To complete the present description, and for the purpose of helping to make the features of the invention more readily understandable, this specification is accompanied by figures constituting an integral part of the same, which by way of illustration and not limitation represent the following.

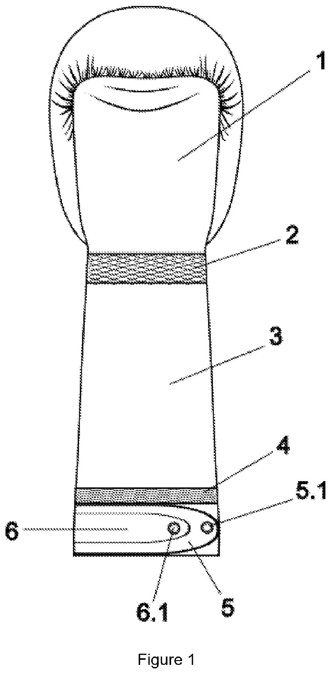

Figure 1 shows a perspective front view of the glove for patients.

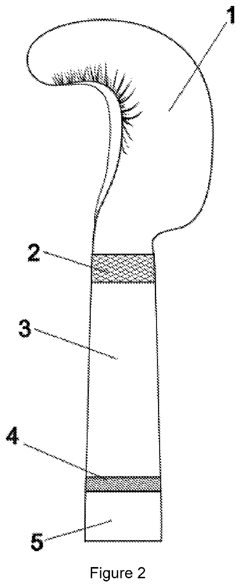

Figure 2 shows a perspective side view of the glove for patients.

### PREFERRED EMBODIMENT OF THE INVENTION

The glove for patients comprises a mitt (1) that completely covers the fingers and hand, extending up to the wrist, where it is attached to a flexible thick strip (2) which is in turn connected to a sleeve (3). There is a narrow strip (4), also flexible, at the upper end of the sleeve (3), said narrow strip being attached to an adjustable fastening strap (5) which has a superimposed securing tape (6) to securely fasten and secure the medical line of the patient, preventing it from being accidentally moved or displaced.

In a preferred embodiment, the mitt (1) is anatomically shaped, lightweight, with a thickly padded back area that has a convex shape on the outside, similar to boxing gloves, while the palm area is thin and has a slightly concave shape, allowing the palm of the hand to rest comfortably. The area for the fingers may have seams that define interdigital areas, providing individual spaces for each finger.

Preferably, the mitt (1) is made of soft, hypoallergenic, padded and breathable materials.

Generally, the thick strip (2) covers part of the contour of the wrist and is made of a very flexible, soft and rubberised material that allows the wrist to move.

In another preferred embodiment, the sleeve (3) has an elongated, tapered configuration, covering the forearm until it reaches near the elbow. The inside is made of a material such as siliconised hollow fibre, while the outside is made of a material such as perforated polyester with elasticity.

Generally, the narrow strip (4) covers the contour near the elbow and is made of a highly flexible, soft and rubberised material that allows the forearm to move.

Generally, the adjustable fastening strap (5) is a hook-and-loop fastening strap that adjusts to the arm and has a first press stud (5.1) for more secure closing.

Preferably, the securing tape (6) is a hook-and-loop joining belt which is secured with a second press stud (6.1) for more secure fastening of the medical lines.

Preferably, the mitt (1), the thick strip (2), the sleeve (3), the narrow strip (4), the adjustable fastening strap (5) and the securing tape (6) are sewn together.

In a preferred embodiment, the inside of the glove has a shell into which the hand is inserted, said shell being rigid or semi-rigid and preventing the fingers from gripping. This shell will allow the fingers to move laterally so that it is more comfortable to use, but not the gripping movement.

This shell will usually have perspiration holes, so that the hand can perspire properly and excessive sweating does not occur.

Having sufficiently described the nature of the present invention, as well as the way of implementing it, it is not considered necessary to extend its explanation for any person skilled in the art to understand its scope and the advantages which derive from it, specifying that, within its essential nature, it can be carried out in other embodiments that differ in detail from the one provided by way of example, and which are also covered by the requested protection, provided that they do not alter, change or modify its fundamental principle.

## Claims

1. A glove for patients, **characterised by** comprising a mitt (1) that completely covers the fingers and hand, extending up to the wrist, where it is attached to a flexible thick strip (2) connected to a sleeve (3), such that there is a flexible narrow strip (4) at the free end of the sleeve (3), said narrow strip being attached to an adjustable fastening strap (5) which has a superimposed securing tape (6) configured to securely fasten and secure the medical line of the patient.

2. The glove for patients according to claim 1, **characterised in that** the mitt (1) is padded.

3. The glove for patients according to claim 1, **characterised in that** the mitt (1) has seams that define individual spaces for each finger.

4. The glove for patients according to claim 1, **characterised in that** the sleeve (3) is tapered.

5. The glove for patients according to claim 1, **characterised in that** the adjustable fastening strap (5) is a hook-and-loop fastening strap that adjusts to the arm and has a first press stud (5.1).

6. The glove for patients according to claim 5, **characterised in that** the securing tape (6) is a hook-and-loop joining belt which is secured with a second press stud (6.1).

7. The glove for patients according to claim 1, **characterised in that** the mitt (1), the thick strip (2), the sleeve (3), the narrow strip (4), the adjustable fastening strap (5) and the securing tape (6) are sewn together.

8. The glove for patients according to claim 1, **characterised in that** the inside thereof comprises a shell into which the hand is inserted, said shell being rigid or semi-rigid and preventing the fingers from gripping.

9. The glove for patients according to claim 8, **characterised in that** the shell has perspiration holes.
